(19) Europäisches Patentamt
European Patent Office
Office européen des brevets

(11) Veröffentlichungsnummer: **0 255 887**
**B1**

# (12) EUROPÄISCHE PATENTSCHRIFT

(45) Veröffentlichungstag der Patentschrift: **29.08.90**

(51) Int. Cl.[5]: **C07C 205/07**, C07C 205/06, C07C 201/06

(21) Anmeldenummer: **87110351.1**

(22) Anmeldetag: **17.07.87**

(54) **Verfahren zur Herstellung von 2.2-Bis (3-nitrophenyl)- hexafluorpropan.**

(30) Priorität: **23.07.86 DE 3624913**

(43) Veröffentlichungstag der Anmeldung:
**17.02.88 Patentblatt 88/7**

(45) Bekanntmachung des Hinweises auf die Patenterteilung:
**29.08.90 Patentblatt 90/35**

(84) Benannte Vertragsstaaten:
**BE CH DE FR GB IT LI NL**

(56) Entgegenhaltungen:
**WO-A-84/02524**
**US-A- 3 310 573**

**CHEMICAL ABSTRACTS, Band 87, Nr. 23, 5. Dezember 1977, Seite 577, Zusammenfassung Nr. 184125v, Columbus, Ohio, US; B.R. LIVSHITS et al.: "Synthesis of hexafluoroisopropylidene-m,m'-diphenyl diisocyanate", & KHIM. PROM-ST. (MOSCOW) 1977, (7), 551-2 " integrated services" 000**

(73) Patentinhaber: **HOECHST AKTIENGESELLSCHAFT, Postfach 80 03 20, D-6230 Frankfurt am Main 80(DE)**

(72) Erfinder: **Schneider, Klaus-Albert, Dr., Schillerring 30, D-6234 Hattersheim am Main(DE)**
Erfinder: **Siegemund, Günter, Dr., Frankfurter Strasse 21, D-6238 Hofheim am Taunus(DE)**

**EP 0 255 887 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

2.2-Bis-(3-nitrophenyl)-hexafluorpropan ist die Verbindung der Formel

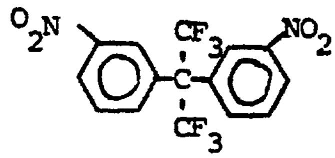

Sie ist hauptsächlich Zwischenprodukt auf dem Polymerengebiet. So kann die Verbindung z.B. zu 2.2-Bis-(3-aminophenyl)-hexafluorpropan reduziert und die Bis-Amino-Verbindung mit aromatischen Tetracarbonsäuren oder deren Anhydriden zu wertvollen Polyimiden mit hoher chemischer und thermischer Beständigkeit kondensiert werden. Ein beispielhaftes Formelschema für eine derartige Kondensation ist:

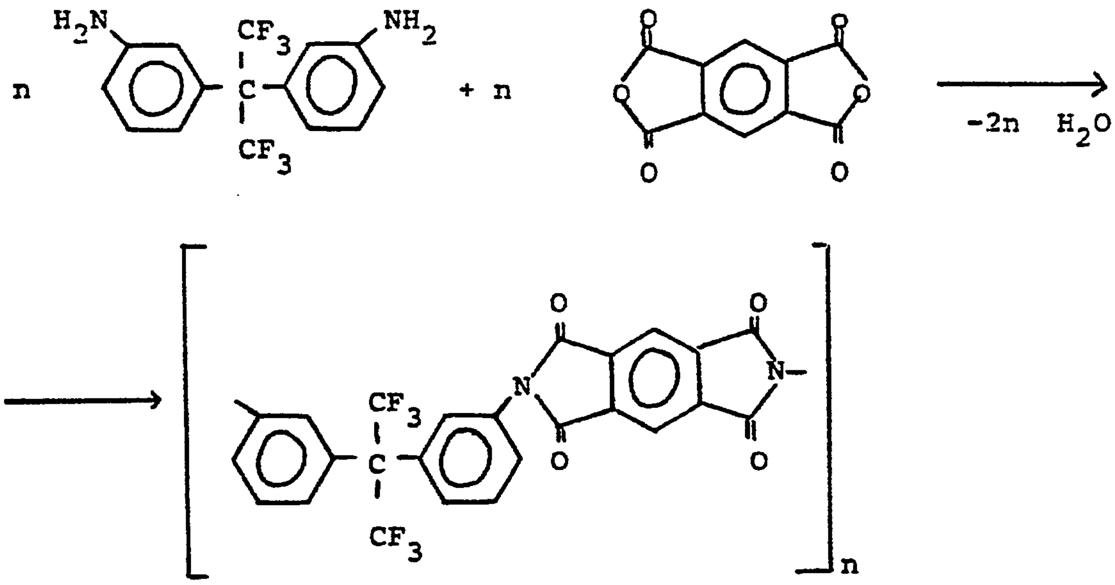


Es ist bekannt, daß das 2.2-Bis-(3-nitrophenyl)-hexafluorpropan in einem 3-Stufen-Verfahren, ausgehend von 2.2-Bis-(4-hydroxyphenyl)-hexafluorpropan hergestellt werden kann (K.S.Y. Lau et al., Journal of Polymer Science, Polymer Chemistry Edition, Vol. 20, S. 238l-2393 (l982)). In der l. Stufe dieses Verfahrens wird die Ausgangsverbindung mit Trifluormethansulfonsäureanhydrid zu 2.2-Bis-(triflatophenyl)-hexafluorpropan umgesetzt;
in der 2. Stufe wird die letztgenannte Verbindung katalytisch zu 2.2-Bisphenylhexafluorpropan hydriert und in der 3. Stufe wird das 2.2-Bisphenylhexafluorpropan mit $HNO_3/H_2SO_4$ zu der Endverbindung 2.2-Bis-(3-nitrophenyl)-hexafluorpropan nitriert.

Formelmäßig stellt sich das Verfahren wie folgt dar:

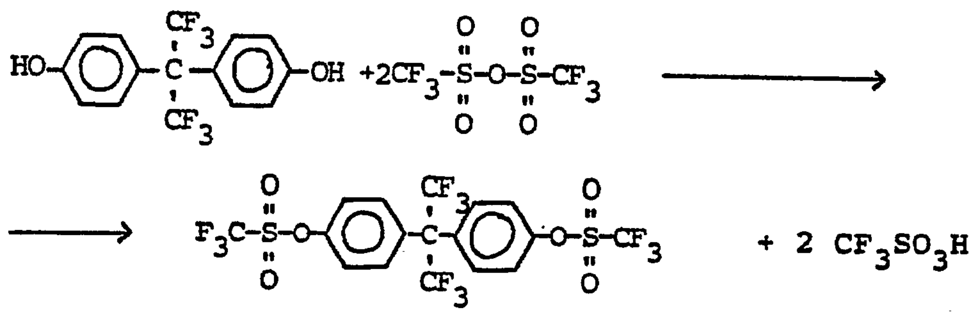

**2. Stufe:**

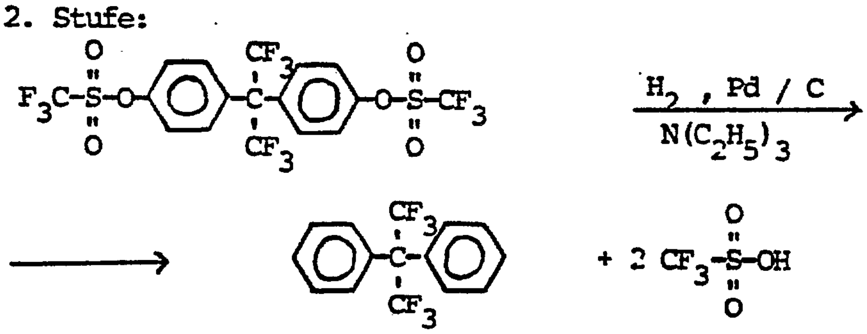


**3. Stufe:**

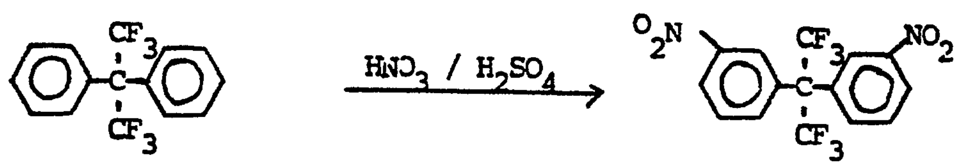


Die Ausgangsverbindung für das Verfahren - 2.2-Bis-(4-hydroxyphenyl)-hexafluorpropan - kann z.B. durch Umsetzung von Hexafluoraceton mit Phenol in flüssigem Fluorwasserstoff erhalten werden (EP-B-0054227).

Nach den Ausführungsbeispielen der vorerwähnten Literaturstelle betragen die Ausbeuten

in der I. Stufe: 96,5 %,
in der 2. Stufe: 87,6 % und
in der 3. Stufe: 90 % d.Th.

Trotz der hohen Ausbeuten ist für das Verfahren nachteilig, daß in der I. Stufe das sehr teure und nur unter erheblichen Sicherheitvorkehrungen handhabbare Trifluorsulfonsäureanhydrid eingesetzt werden muß und daß in der 3. Stufe die Bildung gerirger Anteile der (unerwünschten) 4-Nitro-Isomeren nur mit besonderem Aufwand vermieden oder verringert werden kann. Das Verfahren ist daher insbesondere für die technische Durchführung nur wenig geeignet.

In dem Bestreben, ein verbessertes und vor allem auch technisch einfach durchführbares Verfahren zur Herstellung von 2.2-Bis-(3-nitrophenyl)-hexafluorpropan bereitzustellen, wurde nun gefunden, daß dieses Ziel dadurch erreicht werden kann, daß man von 2.2-Bis-(4-methylphenyl)-hexafluorpropan ausgeht, diese Ausgangsverbindung zu 2.2-Bis-(4-carboxyphenyl)-hexafluorpropan oxidiert, die letztgenannte Verbindung zu 2,2-Bis-(4-carboxy-3-nitrophenyl)-hexafluorpropan nitriert und diese Nitroverbindung dann schließlich decarboxyliert.

Erfindungsgegenstand ist daher ein Verfahren zur Herstellung von 2.2-Bis-(3-nitrophenyl)-hexafluorpropan, das dadurch gekennzeichnet ist, daß man

a) 2.2-Bis-(4-methylphenyl)-hexafluorpropan zu 2.2-Bis-(4-carboxyphenyl)-hexafluorpropan oxidiert,
b) das 2.2-Bis-(4-carboxyphenyl)-hexafluorpropan dann zu 2.2-Bis-(4-carboxy-3-nitrophenyl)-hexafluorpropan nitriert und schließlich
c) das 2.2-Bis-(4-carboxy-3-nitrophenyl)-hexafuorpropan decarboxyliert.

Formelmäßig läßt sich das Verfahren wie folgt wiedergeben:

a) 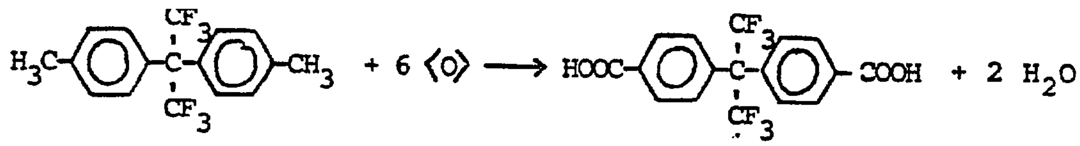

b) 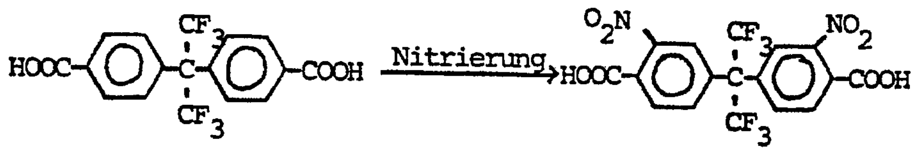


c) 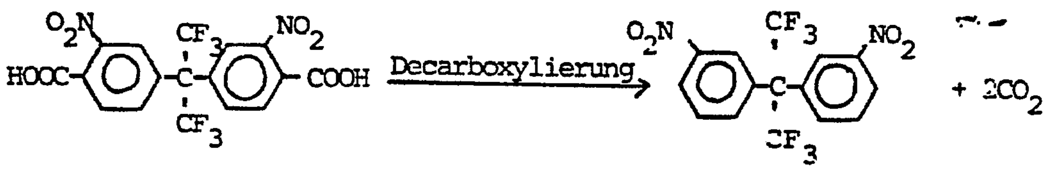


Die Ausbeuten in den einzelnen Verfahrensstufen - und damit auch des Gesamtverfahrens - liegen in der gleichen Größenordnung wie die Ausbeuten der einzelnen Stufen - und des Gesamtverfahrens - gemäß der Literaturstelle von K.S.Y. Lau et al. (a.a.O.). Das erfindungsgemäße Verfahren erfordert jedoch nicht den Einsatz des teuren und gefährlichen Trifluormethylsulfonsäureanhydrids; das Verfahren ist vielmehr mit durchweg gängigen und auch technisch gut beherrschbaren Chemikalien durchführbar. Außerdem besteht hier nicht die Gefahr der Bildung unerwünschter 4-Nitro-Isomerer, weil bei der Nitrierung (in Stufe b) die 4-Stellung der Phenylkerne durch die COOH-Gruppe besetzt ist. Die Verfahrens-Endprodukte sind daher absolut isomerenrein.

Die Verfahrensstufen a), b) und c) sind zwar einzeln - für sich genommen - bekannt oder zu bekannten Verfahren analog; ihre Kombination ist jedoch neu und war durch nichts nahegelegt. Aufgrund des - immerhin ziemlich neuen, aus dem Jahr 1982 stammenden - Standes der Technik gemäß K.S. Y. Lau et al. (a.a.O.) mußte man annehmen, daß 2.2.-Bis-(3-nitrophenyl)-hexafluorpropan nur in ziemlich aufwendiger Weise unter Einsatz teurer und gefährlicher Chemikalien und über ziemlich komplizierte Zwischenstufen (2.2-Bis-(4-triflatophenyl)-hexafluorpropan) herstellbar ist , nicht aber in so einfacher und technisch vorteilhafter Weise wie nach dem erfindungsgemäßen Verfahren.

Die Ausgangsverbindung für das erfindungsgemäße Verfahren - 2.2-Bis-(4-methylphenyl)-hexafluorpropan - ist nach bekannten Methoden erhältlich, z.B. durch Umsetzung von Hexafluoraceton mit Toluol in flüssigem Fluorwasserstoff (B.L. Livsic et al., Z. vses. chim. Obsc. II (1966) Nr. 4, S. 469-470).

Die Oxidation der Ausgangsverbindung 2.2-Bis-(4-methylphenyl)-hexafluorpropan gemäß Verfahrensstufe a) ist eine bekannte Reaktion. Nach B.L. Livsic et al. (a.a.O.) wird diese Oxidation mit 20 %iger Salpetersäure durchgeführt und ergibt die entsprechende Dicarbonsäure - 2.2-Bis-(4-carboxyphenyl)-hexafluorpropan - mit einer Ausbeute von 82 %.

Nach US-A-3 310 573 erfolgt diese Oxidation mit $CrO_3$.

Auch andere von der Herstellung aromatischer Carbonsäuren durch Oxidation von Methylbenzolen her bekannte Methoden sind im vorliegenden Fall gangbar. Bevorzugt ist hier jedoch die Methode der Oxidation mit Chromtrioxid, wie sie in US-A-3 310 573 beschrieben ist.

Die Nitrierung gemäß Verfahrensstufe b) wird bevorzugt mit Nitriersäure (= conc. $HNO_3$/conc. $H_2SO_4$) in ansonsten für derartige Nitrierungen bekannter Weise durchgeführt.

Der bevorzugte Temperaturbereich für die Nitrierung liegt bei etwa 40 bis 100°C, insbesondere bei etwa 70 bis 90°C.

Die Decarboxylierung gemäß Verfahrensstufe c) erfolgt im Prinzip ebenfalls auf bekannte Weise , d.h. wie es von der Decarboylierung anderer aromatischer Carbonsäuren her bekannt ist. Bevorzugt wird die Decarboxylierung im vorliegenden Fall nach der in

Fieser & Fieser, Lehrbuch der organischen Chemie, 4. Aufl. (1960), S. 649, Abs. 2

beschriebenen Methode durchgeführt. Die Methode besteht darin, daß man die aromatische Carbonsäure mit einem Kupferkatalysator in Chinolin gelöst erhitzt. Im vorliegenden Fall ist es allerdings noch günstiger, anstelle des Chinolins Pyridin zu verwenden.

Das Pyridin kann gegebenenfalls auch in Mischung mit anderen schwerflüchtigen organischen Stickstoffbasen eingesetzt werden. Auch der Einsatz nur der anderen schwerflüchtigen organischen Stickstoffbasen - alleine oder in Mischung miteinander - ist möglich.

Als solche anderen schwerflüchtigen organischen Stickstoffbasen kommen - außer dem bereits genannten Chinolin - z.B. in Frage:
die Cl- und Methylderivate des Pyridins und Chinolins; Isochinolin, Chinoxalin, deren Cl- und Methylderivate, etc. Die Stickstoffbasen können - außer als Lösungsmittel - auch in einer für die Lösung der Ausgangsverbindung nicht ausreichenden Menge verwendet werden.

Außerdem ist bei der Decarboxylierung die Gegenwart katalytisch wirkender Substanzen vorteilhaft. Bevorzugt ist die Gegenwart eines Kupferkatalysators, insbesondere von feinteiligem Kupferpulver, gegebenenfalls in Gegenwart von Kupfer-Chrom-Oxid $CuCr_2O_4$ mit einem noch geringen Mangangehalt (insbesondere um etwa 2 % Mn).

Die Menge des Kupferkatalysators ist im Prinzip nicht kritisch; zweckmäßige Mengen liegen bei etwa 0,5 bis 5 Gew.-%, bezogen auf das 2.2-Bis-(4-carboxy-3-nitrophenyl)-hexafluorpropan.

Die Decarboxylierung beginnt bei Temperaturen ab etwa 100°C; höhere Temperaturen z.B. bis etwa 250°C sind möglich. Im allgemeinen wird in der Nähe des Siedepunktes der eingesetzten Stickstoffbase gearbeitet, insbesondere - da Pyridin als Stickstoffbase bevorzugt ist - beim Siedepunkt des Pyridins (115°C).

Die Aufarbeitung erfolgt auf an sich bekannte Weise.

Die beim erfindungsgemäßen Verfahren als Zwischenstufe auftretende Verbindung 2.2-Bis-(4-carboxy-3-nitrophenyl)-hexafluorpropan ist neu. Sie ermöglicht über das erfindungsgemäße Verfahren auf einem insgesamt fortschrittlichen und vorteilhaften Weg die Herstellung der bekannten Verbindung 2.2-Bis-(3-nitrophenyl)-hexafluorpropan sowie der daraus hergestellten bekannten Polymeren.

Die Erfindung wird nun durch das nachfolgende Beispiel näher erläutert.

Beispiel:

a) Oxidation von 2.2-Bis-(4-methylphenyl)-hexafluorpropan:

664 g (2 mol) 2.2-Bis-(4-methylphenyl)-hexafluorpropan wurden in 6000 ml Eisessig bei 80°C portionsweise mit 1500 g (15 mol) Chromtrioxid versetzt. Über Nacht wurde bei 80 - 90°C nachgerührt, dann noch 2 Stunden unter Rückfluß erhitzt. Anschließend wurde der Eisessig im Vakuum weitgehend abgezogen. Dann wurden ca. 3 l Wasser zugesetzt und die Lösung einer Wasserdampf-Destillation unterworfen, um so die gesamte Essigsäure zu entfernen. Nach dem Abkühlen wurde die Reaktionsmischung filtriert und der Filterkuchen mit wenig Wasser gewaschen.

Anschließend wurde der Filterkuchen in heißer Soda-Lösung gelöst und von unlöslichen Resten durch eine Filtration befreit. Durch Ansäuern des Filtrats mit Schwefelsäure wurde die Dicarbonsäure in Freiheit gesetzt. Nach dem Waschen mit wenig Wasser wurden so 621 g 2.2-Bis-(4-carboxyphenyl)-hexafluorpropan (79 % Ausbeute) erhalten.

b) Nitrierung von 2.2-Bis-(4-carboxyphenyl)-hexafluorpropan:

Zu einer aus 720 ml 98 %iger Salpetersäure und 1008 ml konz. Schwefelsäure bestehenden Nitriersäure wurden bei einer Reaktionstemperatur von 70 - 80°C portionsweise 392 g (1 Mol) 2.2-Bis-(4-carboyphenyl)-hexafluorpropan gegeben. Zur Vervollständigung der Reaktion wurde bei dieser Temperatur 3 Stunden nachgerührt. Anschließend wurde die Reaktionsmischung auf Eis gegossen, der Niederschlag abgesaugt und mit eiskaltem Wasser gewaschen. Nach der Trocknung des so erhaltenen Produktes bei 75°C/100 Torr im Trockenschrank wurden 424 g (88 % Ausbeute) analysenreines 2.2-Bis-(4- carboxy-3-nitrophenyl)-hexafluorpropan erhalten. Schmp. 235 - 238°C.

IR (KBr): $\gamma$ = 3350 - 2400 $cm^{-1}$ (COOH), 1720 $cm^{-1}$ (C=O), 1540, 1360 $cm^{-1}$ ($NO_2$), 1305 - 1180 $cm^{-1}$ ($CF_3$).

$^1H$ (DMSO): $\delta$ = 7.9 (mc, 6H, aromat.).

$^{13}C$ (DMSO): $\delta$ = 165.1 (C=O; nicht entkoppelt: dd, $^2J_{C,H}$ = 4 Hz, $^3J_{C,H}$ = 1 Hz), 148.4 (aromt.; nicht entkoppelt: mc), 134.7 (aromat.; nicht entkoppelt: d, $^2J_{C,H}$ = 9 Hz), 134.5 (aromat.; nicht entkoppelt: dd, $^1J_{C,H}$ = 168 Hz, $^2J_{C,H}$ = 7 Hz), 130.9 (aromat.; nicht entkoppelt: d, $^1J_{C,H}$ = 171 Hz), 129.2 (aromat.; nicht entkoppelt: dd, $^2J_{C,H}$ = 5 Hz, $^2J_{C,H}$ =8 Hz), 125.0 (aromat.; dd, $^1J_{C,H}$ = 169 Hz, $^2J_{C,H}$ = 6 Hz), 123.2 (q, $^1J_{C,F}$ = 279 Hz, $CF_3$).

$^{19}F$ (DMSO) $\delta$ = -62.8 ($CF_3$)

$C_{17}H_8F_6N_2O_8$(482.2): Ber.: C 42.3 F 1.7 F 23.6 N 5.8
Gef.: C 42.5 H 1.6 F 23.3 N 5.9

c) Decarboxylierung von 2.2-Bis-(4-carboxy-3-nitrophenyl)-hexafluorpropan

Ein aus 724 g (1.5 Mol) 2.2-Bis-(4-carboxy-3-nitrophenyl)-hexafluorpropan, 2100 ml Pyridin, 20 g Cu-Pulver und 10 g $CuCr_2O_4$ + 2 % Mn bestehendes Reaktionsgemisch wurde so lange unter Rückfluß erhitzt, bis IR-spektroskopisch kein Reaktions-$CO_2$ in der Abluft nachweisbar war.

Anschließend wurde das Reaktionsgemisch durch eine Filtration von den festen Bestandteilen getrennt und vom Filtrat der größte Teil des Pyridins mittels einer Vakuum-Destillation entfernt. Der verbleibende Rückstand wurde in $CH_2Cl_2$ gelöst und zum Entfernen des Rest-Pyridins mit halbkonzentrierter Salzsäure versetzt. Nach dem Abtrennen der wäßrigen Phase und dem Waschen der organischen Phase bis zum Neutralpunkt wurde letztere über $MgSO_4$ getrocknet. Nach Abziehen des Lösungsmittels und Umkristallisation des so erhaltenen Rohproduktes aus Ethanol wurden 463 g (78 % Ausbeute) der Dinitroverbindung erhalten.

Schmp. II6 - II9°C.

IR (KBr): $\gamma$ = I550, I360 $cm^{-1}$ ($NO_2$), I3I0 - II60 $cm^{-1}$ ($CF_3$).

$^1H$ ($CDCl_3$):$\delta$ = 8.3 (mc, 4H, aromt.), 7.7 (mc, 4H, aromat.).

$^{13}C$ ($CDCl_3$):$\delta$= I48.5, I35.6, I34.3, I29.9, I25.I, I24.8 (aromat.), I22.6 (q, $^1J_{C,F}$ = 298 Hz, 2 $CF_3$), 64.6 (mc, $\underline{C}(CF_3)_2$).

$^{19}F$ (DMSO): $\delta$= -64.2 ($CF_3$).

$C_{15}H_8F_6N_2O_4$(394.2): Ber.: C 45.7 H 2.0 F 28.9 N 7.I O I6.2 Gef.: C 45.8 H 2.2 F 28.6 N 7.I O I6.0

**Patentansprüche**

I. Verfahren zur Herstellung von 2.2-Bis-(3-nitrophenyl)-hexafluorpropan,
dadurch gekennzeichnet, daß man

a) 2.2-Bis-(4-methylphenyl)-hexafluorpropan zu 2.2-Bis(4-carboxyphenyl)-hexafluorpropan oxidiert,

b) das 2.2-Bis-(4-carboxyphenyl)-hexafluorpropan dann zu 2.2-Bis-(4-carboxy-3-nitrophenyl)-hexafluorpropan nitriert und schließlich

c) das 2.2-Bis-(4-carboxy-3-nitrophenyl)-hexafluoropropan decarboxyliert.

2. Verfahren nach Anspruch I, dadurch gekennzeichnet, daß man die Oxidation in Stufe a) mit Chromtrioxid durchführt.

3. Verfahren nach den Ansprüchen I bis 2, dadurch gekennzeichnet, daß man die Nitrierung in Stufe b) mit Nitriersäure bei Temperaturen zwischen etwa 40 und I00°C, vorzugsweise zwischen etwa 70 und 90°C, durchführt.

4. Verfahren nach den Ansprüchen I bis 3, dadurch gekennzeichnet, daß man die Decarboxylierung in Stufe c) in Gegenwart schwer flüchtiger organischer Stickstoffbasen sowie eines Kupferkatalysators bei Temperaturen von etwa I00 - 250°C,vorzugsweise bei der Siedetemperatur der verwendeten Stickstoffbasen, durchgeführt.

**Claims**

1. A process for preparing 2,2-bis-(3-nitrophenyl)hexafluoropropane, which comprises

a) oxidizing 2,2-bis-(4-methylphenyl)-hexafluoropropane to 2,2-bis-(4-carboxyphenyl)-hexafluoropropane,

b) then nitrating the 2,2-bis-(4-carboxyphenyl)-hexafluoropropane to 2,2-bis-(4-carboxy-3-nitrophenyl)-hexafluoropropane and finally

c) decarboxylating the 2,2-bis-(4-carboxy-3-nitrophenyl)-hexafluoropropane.

2. The process as claimed in claim 1, wherein the oxidation in stage a) is carried out with chromium trioxide.

3. The process as claimed in claim 1 or 2, wherein the nitration in stage b) is carried out with nitrating acid at temperatures between 40 and 100°C, preferably between 70 and 90°C.

4. The process as claimed in any of claims 1 to 3, wherein the decarboxylation in stage c) is carried out in the presence of organic nitrogen bases of low volatility and of a copper catalyst at temperatures of 100–250°C, preferably at the boiling point of the nitrogen bases used.

**Revendications**

1. Procédé de préparation du bis-(nitro-3 phényl)-2,2 hexafluoropropane, procédé caractérisé en ce que:

a) on oxyde le bis-(méthyl-4 phényl)-2,2 hexafluoropropane de manière à le convertir en le bis-(carboxy-4 phényl)-2,2 hexafluoropropane,

b) on nitre ensuite le bis-(carboxy-4 phényl)-2,2 hexafluoropropane, réaction qui conduit au bis(carboxy-4 nitro-3 phényl)-2,2 hexafluoropropane, et enfin

c) on décarboxyle le bis-(carboxy-4 nitro-3 phényl)-2,2 hexafluoropropane.

2. Procédé selon la revendication 1 caractérisé en ce qu'on effectue l'oxydation, dans l'étape a), au moyen du trioxyde de chrome.

3. Procédé selon l'une des revendications 1 et 2, caractérisé en ce qu'on effectue la nitration, dans l'étape b), au moyen du mélange sulfonitrique, à des températures comprises entre 40 et 100°C, de préférence entre 70 et 90°C.

4. Procédé selon l'une quelconque des revendications 1 à 3, caractérisé en ce qu'on effectue la décarboxylation, dans l'étape c), en présence de bases azotées organiques peu volatiles et d'un catalyseur au cuivre, à des températures comprises entre 100 et 250°C, de préférence à la température d'ébullition des bases azotées utilisées.